# EUROPÄISCHE PATENTSCHRIFT

(11) **EP 0 708 081 B1**
(45) Veröffentlichungstag und Bekanntmachung des Hinweises auf die Patenterteilung: **02.06.1999**
(21) Anmeldenummer: 95113268.7
(22) Anmeldetag: 24.08.1995
(51) Int. Cl.: C07C 249/02, C07C 251/22, C07C 251/24, C07F 7/18

(54) **Verwendung von N,N'-disubstituierten p-Chinondiiminen bei der Herstellung und zur Stabilisierung von Organosilanen**
Use of N,N'-disubstituted p-quinondiimines for preparing and stabilizing organosilanes
Utilisation de diimines de p-quinone N,N'-disubstitué pour la préparation et stabilisation d'organosilanes

(30) Priorität: 21.10.1994 DE 4437667
(43) Veröffentlichungstag der Anmeldung: 24.04.1996
(73) Patentinhaber: HÜLS AKTIENGESELLSCHAFT, 45764 Marl (DE)
(72) Erfinder: Bernhardt, Günther, Dr., D-53757 St Augustin (DE); Steffen, Klaus-Dieter, Dr., D-53773 Hennef (DE); Haas, Margret, D-53639 Königswinter (DE); Kragl, Heinz, D-53844 Troisdorf (DE)
(74) Vertreter: Dörries, Hans, Dr.

(56) Entgegenhaltungen:
- DE-A- 3 832 621
- US-A- 2 118 826
- US-A- 4 722 807
- MONATSH. CHEMIE, Bd. 8, 1887, Seite 478 XP002021561 BANDROWSKY:

## Beschreibung

Die Erfindung betrifft die Verwendung von N,N'-disubstituierten p-Chinondiiminen der allgemeinen Formel I 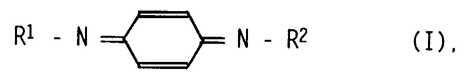 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, zur Stabilisierung von Methacryloxy- oder Acryloxygruppen enthaltenden Organosilanen der allgemeinen Formel II 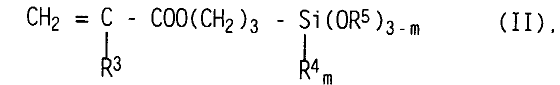 in der R³ für ein Wasserstoffatom oder eine Methyl-Gruppe steht und R⁴ und R⁵ gleiche oder verschiedene Alkyl-Gruppen mit 1 bis 4 C-Atomen sind und m gleich 0 oder 1 oder 2 ist.

Die Organosilane der allgemeinen Formel II werden nachfolgend auch als Acrylsilane bezeichnet.

Die erfindungsgemäß als Stabilisatoren verwendeten N,N'-disubstituierten p-Chinondiimine der allgemeinen Formel I sowie Verfahren zu deren Herstellung sind bekannt. Für diese Verfahren verwendet man als Ausgangsstoffe die korrespondierenden N,N'-disubstituierten p-Phenylendiamine, die unter Einwirkung von Oxidationsmitteln in die entsprechenden Diimine überführt werden. So ist die Herstellung von N,N'-Diphenyl-p-chinondiimin aus dem korrespondierenden Diamin durch Oxidation mit Chromsäure in verdünnter essigsaurer Lösung beschrieben (Chem.Ber. 46 (1913) S. 1853). Es ist weiter bekannt, N,N'-Diphenyl-p-chinondiimin durch Schütteln einer benzolischen Lösung von N,N'-Diphenyl-p-phenylendiamin mit einer wäßrigen Kaliumferricyanid-Lösung herzustellen (F. Feichtmayr u. F. Würstlin, Berichte der Bunsengesellschaft Bd. 67 (1963) S. 435). Das gleiche Verfahren wird auch für die Herstellung von N-Phenyl-N'-isopropyl-p-chinondiimin und N-Phenyl-N'-cyclohexyl-p-chinondiimin beschrieben (L. Kotulak et al., Collect. Czech. Chem. Commun. 48 (1983) 12, S. 3384-3395). Auch die Verwendung von Silberoxid anstelle von Kaliumferricyanid als Oxidationsmittel wurde vorgeschlagen (L. Kotulak et al., Collect. Czech. Chem. Commun. 48 (1983) 12, S. 3384-3395).

Bei anderen bekannten Verfahren dient Luftsauerstoff als Oxidationsmittel. So wird N,N'-Diphenyl-p-phenylendiamin in Anwesenheit von hohen Überschüssen an Azoisobuttersäuredinitril (AIBN) durch Luftsauerstoff in ein Diimin umgewandelt (C. E. Boozer et al., Journ. Amer. Chem. Soc. 77 (1955) S.3233). E.v.Bendrowsky beschreibt in Monatshefte für Chemie, VIII. Band (1887) die Herstellung von N,N'-Diphenyl-p-chinondiimin durch Oxidation von N,N'-Diphenyl-p-phenylendiamin mit Luftsauerstoff in einer siedenden alkoholischen Lösung, die zuvor mit Ätzkali versetzt war. In der US-PS 2 118 826 ist die Oxidation von N,N'-disubstituierten p-Arylendiaminen zu den korrespondierenden p-Chinondiiminen mit Luft in Gegenwart von festen Alkali- oder Erdalkalioxiden, -hydroxiden, -carbonaten oder -amiden beschrieben.

EP-PS 0 437 653 lehrt die Synthese von Organosilanen der allgemeinen Formel II durch Umsetzung von Alkalisalzen der Methacrylsäure oder Acrylsäure mit Chlorpropylsilanen der allgemeinen Formel III, 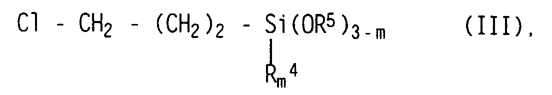 in der R⁴, R⁵ und m die obengenannte Bedeutung haben, in Gegenwart von Phasentransferkatalysatoren des Tetraalkylammoniumtyps, wobei zur Verhinderung unerwünschter Polymerisatbildung, insbesondere der Ausbildung von "Popcorn"-Polymerisat, als Stabilisator N,N'-Diphenyl-p-phenylendiamin in den angeführten Beispielen verwendet wird. Es ist bekannt, daß z.B. Schwermetallsalze die unerwünschte Polymerisation der Acrylsilane auslösen und auch die Siloxanbildung von Alkoxysilanfunktionen beschleunigen können. Die Reindarstellung der Acrylsilane erfolgt durch destillative Maßnahmen.

Die Synthese der Acrylsilane nach dem Verfahren der Phasentransferkatalyse, im folgenden auch als PTC-Verfahren bezeichnet, umfaßt im allgemeinen vier Einzelschritte:
Stufe 1: Herstellung des Alkalimethacrylats oder -acrylats
Stufe 2: Umsetzung dieser Salze mit Chlorpropylalkoxysilanen in Gegenwart des Phasentransferkatalysators zum (Meth)acrylsilan
Stufe 3: Abtrennen des Begleitproduktes Alkalichlorid
Stufe 4: destillative Aufbereitung des Rohproduktes

Der Zusatz des Stabilisatorsystems erfolgt im allgemeinen nach der ersten Verfahrensstufe. der Zubereitung der Alkaliacrylate.

Wenig vorteilhaft im PTC-Verfahren ist, daß der obengenannte Stabilisator in der vierten Stufe, der destillativen Aufbereitung der Roh-Organosilane der allgemeinen Formel II, wegen seiner Schwerflüchtigkeit nicht in die Dampfphase gelängt. Dies kann insbesondere bei kontinuierlicher Prozeßführung in den Destillationskolonnen oder den Rohrsystemen zur Ausbildung von "Popcorn"-Polymerisat führen, wodurch Betriebsstörungen oder auch Schäden an der Anlage resultieren.

In der DE-PS 38 32 621 werden zur Verbesserung der Stabilisierung von Acrylsilanen, die nach dem Verfahren der Addition von Trialkylsilanen an Allylmethacrylat unter Verwendung von H₂PtCl₆ als Katalysator hergestellt werden, Stabilisatorkombinationen aus N,N'-disubstituierten p-Phenylendiaminen und sterisch gehinderten Phenolen offenbart, wobei die Diamine bei der destillativen Aufarbeitung der Roh-Acrylsilane die Stabilisierung des Sumpfes übernehmen und die flüchtigen Phenole die Stabilisierung der Dampfphase. Die technische Durchführung dieses Verfahrens sieht den Zusatz der Stabilisatoren vor oder nach Abschluß der Syntheseschritte vor. Die erreichbaren Ausbeuten an Acrylsilanen sind jedoch unbefriedigend.

Die Stabilisatorkombination aus N,N'-disubstituierten Diaminen und sterisch gehinderten Phenolen erweist sich zudem bei der Durchführung der (Meth)acrylsilan-Synthese nach dem PTC-Verfahren als unwirksam. Dabei wird durch Zusatz der Stabilisatorkombination im Verlaufe oder nach Beendigung der ersten Verfahrensstufe, der verfahrensüblichen Herstellung des Alkalimethacrylats oder -acrylats aus alkoholischer Alkalialkoholat-Lösung und Methacrylsäure oder Acrylsäure, die phenolische Komponente in nicht flüchtiges Alkaliphenolat überführt, und zwar unabhängig davon, ob bei der Salzbereitung die Säure oder das Alkoholat vorgelegt wird. So steht bei der destillativen Aufarbeitung der Roh-Acrylsilane das freie, flüchtige Phenol nicht zur Verfügung, wodurch eine "Popcorn"-Polymerisatbildung in den Kolonnen auftritt und die Produktausbeute sich verschlechtert.

Der Erfindung liegt daher die Aufgabe zugrunde, ein wirtschaftliches und umweltverträgliches Stabilisatorsystem bereitzustellen, das eine Polymerisation der Organosilane der allgemeinen Formel II während des Herstellprozesses, insbesondere auch bei der destillativen Produktaufarbeitung, verhindert.

Dementsprechend ist einer der Gegenstände der Erfindung die Verwendung von N,N'-disubstituierten p-Chinondiiminen der Formel I, 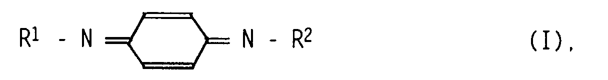 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, zur Stabilisierung von Methacryloxy- oder Acryloxy-Gruppen enthaltenden Organosilanen der allgemeinen Formel II 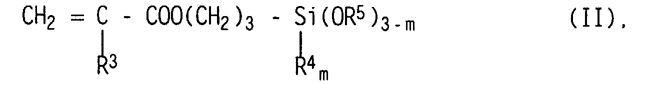 in der R³ für ein Wasserstoffatom oder eine Methyl-Gruppe steht und R⁴ und R⁵ gleiche oder verschiedene Alkyl-Gruppen mit 1 bis 4 C-Atomen sind und m gleich 0 oder 1 oder 2 ist.

Ein anderer Gegenstand der Erfindung sind Zusammensetzungen, bestehend aus Methacryloxy- oder Acryloxy-Gruppen enthaltenden Organosilanen der allgemeinen Formel II 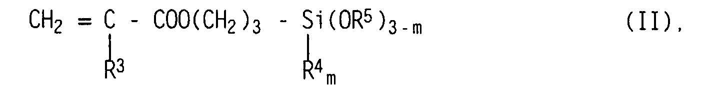 in der R³ für ein Wasserstoffatom oder eine Methyl-Gruppe steht und R⁴ und R⁵ gleiche oder verschiedene Alkyl-Gruppen mit 1 bis 4 C-Atomen sind und m gleich 0 oder 1 oder 2 ist, und einem oder mehreren N,N'-disubstituierten p-Chinondiiminen der allgemeinen Formel I 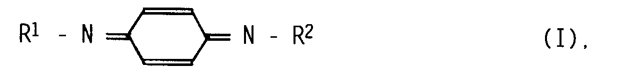 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, als Stabilisator(en) enthalten.

Ferner wurde gefunden, daß N,N'-disubstituierte p-Chinondiimine der allgemeinen Formel I eine Stabilisierung der Acrylsilane während deren Herstellung, insbesondere bei deren destillativer Aufarbeitung - gleichermaßen im Sumpf als auch in der Dampfphase -, bewirken sowie zu verbesserten Ausbeuten führen. Besonders überraschend war, daß N,N'-disubstituierte p-Chinondiimine auch während des PTC-Herstellprozesses durch gezielte Oxidation von N,N'-disubstituierten p-Phenylendiaminen der allgemeinen Formel IV 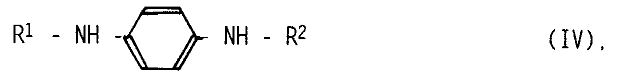 erzeugt werden können, ohne den Prozeßablauf und die Synthese der Acrylsilane ansonsten nachteilig zu beeinflussen. So konnte in einfacher und wirtschaftlicher Weise das PTC-Verfahren noch deutlich verbessert werden.

Dementsprechend ist ein weiterer Gegenstand der vorliegenden Erfindung ein Verfahren zur Stabilisierung von Methacryloxy- oder Acryloxy-Gruppen enthaltenden Organosilanen der allgemeinen Formel II 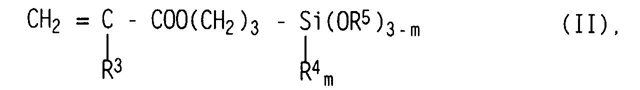 in der R³ für ein Wasserstoffatom oder eine Methyl-Gruppe steht und R⁴ und R⁵ gleiche oder verschiedene Alkyl-Gruppen mit 1 bis 4 C-Atomen sind und m gleich 0 oder 1 oder 2 ist, das dadurch gekennzeichnet ist, daß man als Stabilisatoren N,N'-disubstituierte p-Chinondiimine der allgemeinen Formel I 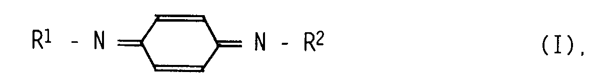 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, einsetzt.

Ein anderer Gegenstand der Erfindung ist ein Phasentransferkatalyse-Verfahren (PTC-Verfahren) zur Herstellung von Methacryloxy- oder Acryloxy-Gruppen enthaltenden Organosilanen der allgemeinen Formel II 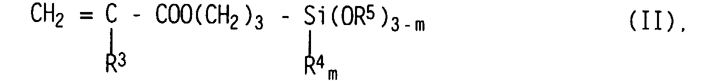 in der R³ für ein Wasserstoffatom oder eine Methyl-Gruppe steht und R⁴ und R⁵ gleiche oder verschiedene Alkyl-Gruppen mit 1 bis 4 C-Atomen sind und m gleich 0 oder 1 oder 2 ist, das dadurch gekennzeichnet ist, daß die Roh-Organosilane der allgemeinen Formel II während des PTC-Verfahrens durch N,N'-disubstituierte p-Chinondiimine der allgemeinen Formel I 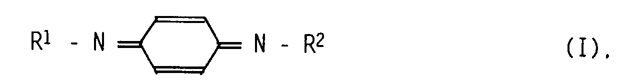 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, stabilisiert werden.

Die für die beschriebenen Zwecke verwendeten p-Chinondiimine, sowie deren Herstellung, sind nicht Gegenstand der Erfindung. Sie können durch z.B. nach dem Verfahren der gleichzeitig anhängigen europäischen Patentanmeldung 95 113 266.1 durch Oxidation von N,N'-disubstituierten p-Phenylendiaminen mittels Halogenen hergestellt werden. Bei einer anderen. vorbekannten Variante dient molekularer Sauerstoff als Oxidationsmittel, und die Oxidation findet in alkalisch-alkoholischer Lösung statt. Dabei können als alkalischer Bestandteil der alkalischalkoholischen Lösung Alkalialkoholate und/oder Alkalihydroxide eingesetzt werden.

Bevorzugt enthält die alkalisch-alkoholische Lösung 5 bis 15 Gew.-% Alkalialkoholat. Es können aber auch höhere oder niedrigere Alkoholat-Konzentrationen verwendet werden, wobei allerdings bei höheren Konzentrationen als 15 Gew.-% in zunehmendem Maße N,N'-disubstituierte 2,5-Dialkoxy-p-chinondiimine als Nebenprodukte entstehen und bei geringerer Konzentration als 5 Gew.-% die Oxidation zunehmend langsamer verläuft. Bevorzugte Alkalialkoholate sind die Methylate und Ethylate des Natriums und Kaliums.

Als Alkohole können hier z.B. primäre, sekundäre oder tertiäre Alkohole mit 1 bis 6 C-Atomen im Kohlenstoffgerüst verwendet werden, bevorzugt sind Methanol und Ethanol.

Für die Oxidation kann das eingesetzte Diamin der allgemeinen Formel IV vollkommen oder auch nur teilweise in der verwendeten Alkoholat-Lösung gelöst vorliegen.

Bevorzugt wird eine schnelle Oxidation, um die Bildung von Nebenprodukten gering zu halten. Die Oxidation wird vorzugsweise bei einer Temperatur zwischen 10 und 60°C, besonders vorzugsweise bei einer Temperatur zwischen 20 und 40°C, ganz besonders vorzugsweise bei einer Temperatur zwischen 25 und 35°C, durchgeführt.

Als Oxidationsmittel kann molekularer Sauerstoff verwendet werden. Bevorzugt wird als Oxidationsmittel Sauerstoff im Gemisch mit Stickstoff verwendet. Dabei wird als Oxidationsmittel ein Sauerstoff/Stickstoff-Gemisch, vorzugsweise mit einem Gehalt von mehr als 0,1 und weniger als 21 Vol.-% Sauerstoff, besonders vorzugsweise von mehr als 0,1 und weniger als 8,4 Vol.-% Sauerstoff, verwendet. Bevorzugt wird drucklos gearbeitet. Es kann aber auch bei höheren Drücken gearbeitet werden, geeigneterweise bis 5 bar.

Die erfindungsgemäß eingesetzten Diimine brauchen nicht in reiner Form vorzuliegen. So kann das Produkt der Oxidationsreaktion ein Gemisch von N,N'-disubtituiertem p-Phenylendiamin und N,N'-disubstituiertem p-Chinondiimin enthalten. Schon bei einem 10 %igen Umsatz des Diamins zum Diimin kann die Zugabe zu Organosilanen der allgemeinen Formel II stabilisierend wirken, jedoch sollte der Diamin-Umsatz mehr als 30 % beträgen.

Die wie beschrieben hergestellten Diimine können durch gebräuchliche Aufarbeitungsmethoden, wie z. B. destillative Aufarbeitung unter vermindertem Druck, Umkristallisation oder Säulenchromatografie, in reiner Form dargestellt werden. Der Einsatz in analysenreiner Form ist aber, wie bereits erwähnt, keine Vorbedingung für die erfindungsgemäße Verwendung der Diimine. Für die erfindungsgemäße Verwendung zur Stabilisierung der Organosilane der allgemeinen Formel II beträgt deren Gehalt an p-Chinondiiminen der allgemeinen Formel I vorzugsweise > 0,0005 bis 1,5 Gew.-%, besonders vorzugsweise 0,01 bis 1,0 Gew.-%, ganz besonders vorzugsweise 0,1 bis 0,5 Gew.-%.

Die N,N'-disubstituierten p-Chinondiimine können zur Stabilisierung der Organosilane der Formel II verwendet werden. Als zusätzliche Stabilisatoren können die erfindungsgemäß stabilisierten Organosilane N,N'-disubstituierte p-Phenylendiamine der allgemeinen Formel IV 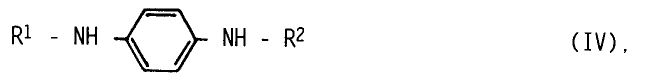 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, und/oder flüchtige, sterisch gehinderte Phenole der allgemeinen Formel V 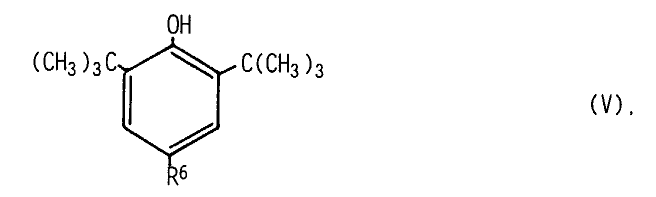 worin R⁶ eine Alkyl- oder Alkoxy-Gruppe mit 1 bis 4 C-Atomen ist, enthalten.

Weiterhin werden die N,N'-disubstitierten p-Chinondiimine I erfindungsgemäß als Stabilisatoren in einem Verfahren zur Herstellung von Methacryloxy- oder Acryloxy-Gruppen enthaltenden Organosilanen der allgemeinen Formel II verwendet. In dem eingangs dargestellten PTC-Verfahren werden Organosilane der allgemeinen Formel II aus Alkalimethacrylaten oder Alkaliacrylaten und Chlorpropylalkoxysilanen der allgemeinen Formel III 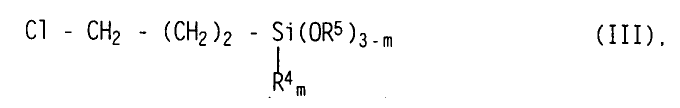 in der R⁴ und R⁵ für gleiche oder verschiedene Alkyl-Gruppen mit 1 bis 4 C-Atomen und m für 0 oder 1 oder 2 stehen, erhalten.

Bei dem erfindungsgemäßen Verfahren zur Stabilisierung von Methacryloxy- oder Acryloxy-Gruppen enthaltenden Organosilanen der allgemeinen Formel II können die als Stabilisatoren eingesetzten p-Chinondiimine der allgemeinen Formel I während des Herstellprozesses der Organosilane der allgemeinen Formel II zugesetzt werden.

Die als Stabilisatoren eingesetzten p-Chinondiimine der allgemeinen Formel I können aber auch während des Herstellprozesses der Organosilane der allgemeinen Formel II erzeugt werden. Dabei können die p-Chinondiimine der allgemeinen Formel I durch Oxidation der korrespondierenden p-Phenylendiamine der allgemeinen Formel IV erzeugt werden. Als Oxidationsmittel wird vorzugsweise Sauerstoff im Gemisch mit Stickstoff verwendet. Die Oxidation wird vorzugsweise bei einer Temperatur zwischen 10 und 60°C, besonders vorzugsweise bei einer Temperatur zwischen 20 und 40°C, ganz besonders vorzugsweise bei einer Temperatur zwischen 25 und 35°C, durchgeführt.

Die p-Chinondiimine der allgemeinen Formel I können so z.B. durch Oxidation der korrespondierenden p-Phenylendiamine der allgemeinen Formel IV im Reaktionsmilieu der Stufe 1 des PTC-Verfahrens erzeugt werden.

Man kann so verfahren, daß zunächst die vorgelegte Alkalialkoholat-Menge durch Zugabe von Methacrylsäure oder Acrylsäure soweit neutralisiert wird, daß in einer z.B. methanolischen Lösung nach Abzug der resultierenden Salzmenge der Gewichtsanteil des verbleibenden, gelösten Alkalialkoholats zwischen 5 und 15 Gew.-% beträgt. Nach dem Einbringen eines N,N'-disubstituierten Diamins der allgemeinen Formel IV kann durch die gezielte O₂-Oxidation der gewünschte Umsatz des Diamins zum Diimin herbeigeführt werden. Der als Oxidationsmittel verwendete molekulare Sauerstoff kann durch ein Inertgas, vorzugsweise Stickstoff, soweit verdünnt werden, daß mit keiner der im obengenannten System befindlichen Komponenten explosionsfähige Gemische entstehen. Anschließend kann für eine stöchiometrische Salzbildung weitere Säure hinzugefügt werden. Es kann aber auch ein geringer Alkoholat-Überschuß verbleiben, so daß eine mit Wasser im Verhältnis 1:1 gemischte, homogenisierte alkoholische Salzprobe einen pH-Wert zwischen 8 und 11 erreicht. In diesem alkalisch/alkoholischen Milieu erwiesen sich die erfindungsgemäß hergestellten Diimine auch über einen längeren Zeitraum als stabil. Nach Durchführung der phasentransferkatalysierten Reaktion in Stufe 2 bzw. nach der Salzabtrennung in Stufe 3 liegt nunmehr das Roh-Organosilan der allgemeinen Formel II vor. Die erfindungsgemäß hier stabilisierten Roh-Organosilane der allgemeinen Formel II enthalten vorzugsweise >0,0005 bis 1,5 Gew.-%, besonders vorzugsweise 0,01 bis 1,0 Gew.-%, ganz besonders vorzugsweise 0,1 bis 0,5 Gew.-% p-Chinondiimine der allgemeinen Formel I. Bevorzugte N,N'-disubstituierte p-Chinondiimine sind solche, die eine Flüchtigkeit aufweisen. die nicht höher liegt als der Siedepunkt des zu stabilisierenden Acrylsilans. Vorzugsweise liegen die Flüchtigkeiten der Diimine nur unwesentlich unter den Siedepunkten der Acrylsilane.

Wie die Praxis zeigt, können während des PTC-Verfahrens die als Stabilisatoren verwendeten p-Chinondiimine der allgemeinen Formel I zugesetzt und/oder erzeugt werden.

Man kann aber auch für das erfindungsgemäße Verfahren zur Stabilisierung der Organosilane der allgemeinen Formel II als zusätzliche Stabilisatoren N,N'-disubstituierte p-Phenylendiamine der allgemeinen Formel IV 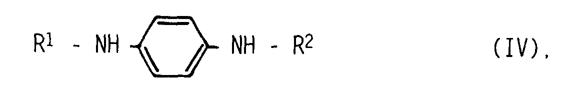 worin R¹ und R¹ gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, und/oder flüchtige, sterisch gehinderte Phenole der allgemeinen Formel V 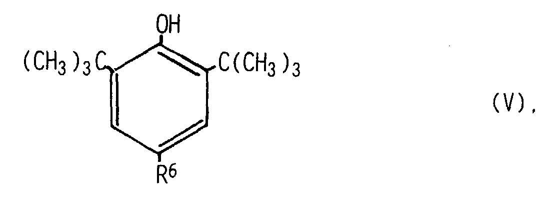 worin R⁶ eine Alkyl- oder Alkoxy-Gruppe mit 1 bis 4 C-Atomen ist, einsetzen. Geeigneterweise weisen die phenolischen Stabilisatoren vergleichbare Siedepunkte wie die Acrylsilane der allgemeinen Formel II auf.

Die Reindarstellung der Organosilane der allgemeinen Formel II erfolgt üblicherweise durch destillative Maßnahmen. Sie können unter Verwendung von Kurzweg- oder Dünnschichtverdampfern und/oder durch Kolonnendestillation erfolgen.

Die Roh-Organosilane der allgemeinen Formel II, die p-Chinondiimine der allgemeinen Formel I enthalten, werden zur Abtrennung von Hochsiedern vorzugsweise einer Flash-Destillation unterzogen. Unter Flash-Destillation versteht man eine destillative Vorreinigung, beispielsweise über einen Dünnschichtverdampfer.

Eine bevorzugte Ausführungsform der destillativen Aufbereitung der rohen Organosilane II besteht in der Kombination von Flash-Destillation (Vorstufe) und Kolonnendestillation. In der Vorstufe können Katalysatoren, nicht umgesetzte Diamine, Restsalze und geringe Mengen Polymerisat abgetrennt werden.

Das Flash-Destillat wird mit einer Menge von vorzugsweise >5 bis 15.000 Gew.-ppm, besonders vorzugsweise 10 bis 3.000 Gew.-ppm und ganz besonders vorzugsweise 50 bis 1.000 Gew.-ppm, p-Chinondiiminen der allgemeinen Formel I stabilisiert.

Nachfolgend wird das Flash-Destillat geeigneterweise fraktioniert destilliert.

Ein Vorteil der erfindungsgemäß verwendeten p-Chinondiimine I besteht darin, daß sie eine hohe Flüchtigkeit aufweisen und bei der Destillation der Acrylsilane auch in die Dampfphase gelangen und dort stabilisierend, d.h. polymerisationsverhindernd, wirken. Die dabei über Kopf der Kolonne destillierten Methacryloxy- oder Acryloxy-Gruppen enthaltenden Organosilane werden vorzugsweise frei von p-Chinondiiminen der allgemeinen Formel I erhalten.

Den Methacryloxy- oder Acryloxy-Gruppen enthaltenden Organosilanen der allgemeinen Formel II wird vorzugsweise nach der fraktionierten Destillation ein nicht färbendes, sterisch gehindertes Phenol der allgemeinen Formel V zur Nachstabilisierung zugegeben; bevorzugt wird 2,6-Di-tert.-butyl-4-methylphenol zugegeben. Die destillative Aufarbeitung ist sowohl batchweise als auch kontinuierlich durchführbar.

Die Konzentrationsbestimmung der N,N'-disubstituierten Diimine erfolgt geeigneterweise photometrisch.

Im erfindungsgemäßen PTC-Verfahren können die p-Chinondiimine der allgemeinen Formel I und N,N'-disubstituierte p-Phenylendiamine der allgemeinen Formel IV dem Eduktgemisch und/oder dem Reaktionsgemisch und/oder dem Roh-Organosilan der allgemeinen Formel II und/oder dem Flash-Destillat und/oder an einer anderen Stelle der destillativen Aufarbeitung zugegeben werden; darüber hinaus können dem Roh-Organosilan der allgemeinen Formel II und/oder dem Flash-Destillat und/oder an einer anderen Stelle der destillativen Aufarbeitung und/oder dem Reindestillat flüchtige, sterisch gehindert Phenole der allgemeinen Formel V zugegeben werden.

Alle Destillationen werden geeigneterweise unter vermindertem Druck durchgeführt, die bevorzugten Arbeitsdrücke liegen zwischen 0,01 und 50 mbar.

Geeignete N,N'-disubstituierte p-Chinondiimine I sind z. B.:
N,N'-Diphenyl -p-chinondiimin,
N,N'-Di-(1-methylheptyl)-p-chinondiimin,
N,N'-Di-(1-ethyl-3-methylpentyl)-p-chinondiimin,
N,N'-Di-(1,4-dimethylpentyl)-p-chinondiimin,
N,N'-Di-sek.-butyl-p-chinondiimin,
N-Phenyl-N'-cyclohexyl-p-chinondiimin,
N-Phenyl-N'-isopropyl-chinondiimin

Geeignete N,N'-disubstituierte p-Phenylendiamine II sind z. B.:
N,N'-Diphenyl-p-phenylendiamin,
N,N'-dinaphthyl-p-phenylendiamin,
N,N'-Di-(1-methylheptyl)-p-phenylendiamin,
N,N'-Di-(1-ethyl-3-methylpentyl)-p-phenylendiamin,
N,N'-Di-(1,4-dimethylpentyl)-p-phenylendiamin,
N,N'-Di-sek.-butyl-p-phenylendiamin,
N-Phenyl-N'-cyclohexyl-p-phenylendiamin oder
N-Phenyl-N'-isopropyl -p-phenylendiamin.

Geeignete, sterisch gehinderte Phenole V sind z. B.:
2,6-Di-tert.-butyl-4-methylphenol,
2,6-Di-tert.-butyl-4-ethylphenol,
2,6-Di-tert.-butyl-4-methoxyphenol

Durch die Verwendung von N,N'-disubstituierten p-Chinondiiminen I bei der Herstellung von Methacryloxy- oder Acryloxy-enthaltenden Organosilanen der allgemeinen Formel II, insbesondere im PTC-Verfahren, gelingt es in einfacher Weise, die "Popcorn"-Polymerisation zu verhindern und Acrylsilane von hoher Reinheit und farblosem Aussehen zu erhalten. Vor allem Organosilane der allgemeinen Formel II, die zur Herstellung von Polymerisaten verwendet werden, haben solch hohen Qualitätsanforderungen zu entsprechen.

Ein weiterer wesentlicher Vorteil der erfindungsgemäßen Maßnahmen besteht darin, daß die Ausbeute an spezifikationsgerechten Acrylsilanen II und damit die Wirtschaftlichkeit des PCT-Verfahrens noch weiter verbessert werden konnte.

Die Erfindung wird durch die nachfolgenden Beispiele näher erläutert.

### Beispiele

### Herstellung von N,N'-Diphenyl-p-chinondiimin (kein Beispiel nach der Erfindung)

57 g technisch hergestelltes N,N'-Diphenyl -p-phenylendiamin (Handelsprodukt Permanax DPPD^{(R)} der Fa. Akzo) mit einem Schmelzbereich von 134 bis 140 °C wurde bei 0,1 mbar unter Stickstoff destilliert. Die bei 196 bis 200 °C übergehende Fraktion wurde gesammelt. Ausbeute 44,5 g 78,1 % d. Th.; Schmelzpunkt: 135 - 138 °C. 33 g Destillat wurden aus Aceton/Methanol umkristallisiert. Ausbeute: 82,0 % d. Th.; Schmelzpunkt: 149 - 150 °C.
In eine Lösung von 22 g N,N'-Diphenyl-p-phenylendiamin in 5 1 8 %iger Kaliummethylat/-Methanol-Lösung wurde unter Rühren bei einer Temperatur um 30 °C ein lebhafter Gasstrom aus O₂ und N₂ (1 : 11) eingeleitet. Nach 3 h betrug die N,N'-Diphenyl-p-chinondiimin-Ausbeute 78 %. Die Ausbeute wurde photometrisch aus der Extinktion beim Absorptionsmaximum von 442 nm ermittelt. Der ausgefallene Feststoff wurde abfiltriert. Der Schmelzpunkt wurde nach Umkristallisation aus Aceton/Methanol bestimmt: 181 - 183 °C.

### Beispiel 1

926,1 g einer 25,4 %igen Kaliummethylat-Lösung wurden unter Rühren und Kühlung mit 289,0 g Methacrylsäure versetzt. 4,2 g des wie beschrieben hergestellten N,N'-Diphenyl-p-chinondiimins, 15,2 g Tetrabutylammoniumbromid und 680,3 g 3-Chlorpropyltrimethoxysilan wurden hinzugefügt und über eine kurze Kolonne solange Methanol abdestilliert - ggf. unter Anlegen von Vakuum - bis bei 115 C eine exotherme Reaktion einsetzte. Nach deren Abklingen wurde noch 2 h bei 120°C weitererhitzt. Aus der abgekühlten Mischung wurde das ausgefallene Kaliumchlorid abfiltriert und dieses mit Methanol ausgewaschen. Das vom Methanol befreite Filtrat wurde bei 0,4 mbar über eine Claisenbrücke abgeflasht: Siedepunkt 88-90 °C. Destillatmenge: 766,8 g (92,1 % d.Th.). Das gelb-orange gefärbte Destillat enthielt nach photometrischer Bestimmung 80 mg N,N'-Diphenyl-p-chinondiimin/kg. Durch fraktionierte Destillation über eine mit Raschigringen ausgestatteten Füllkörperkolonne wurden 722,3 g 86,7 % d.Th. 3-Methacryloxypropyltrimethoxysilan erhalten. Siedepunkt: 81-82 °C/0,2 mbar; GC-Reinheit: 99,7 %.
Vor Beginn der Reindestillation wurden in die Destillationsvorlage 144 mg 2,6-Di-tert.-butyl-4-methylphenol (Handelsprodukt Ionol CP der Fa. Shell) eingebracht. Während der Flash- und Reindestillation wurden in die Kolonnensümpfe geringe Mengen eines O₂/N₂-Gemisches mit einem Verhältnis O₂:N₂ von 1:11,5 eingeperlt.
Nach den Destillationen wären in den benutzten Apparaturen, in den Destillaten und Sümpfen keine Hinweise für "Popcorn"-Polymerisate zu finden.

### Beispiel 2

In einem Rührreaktor wurden 13,613 kg einer methanolischen 25,7 %igen Kaliummethylat-Lösung unter Kühlung mit 2,868 kg wasserfreier Methacrylsäure versetzt und nach Zugabe von 62,5 g N,N'-Diphenyl-p-phenylendiamin wurde bei rund 20 bis 30 °C solange ein lebhafter Gasstrom, bestehend aus einem Sauerstoff/Stickstoff-Volumengemisch von 1:12 hindurchgeleitet, bis ein photometrisch ermittelbarer Diamin-Umsatz von 55 % zum N,N'-Diphenyl-p-chinondiimin vorlag. Nach Zugabe von weiteren 1,432 kg Methacrylsäure wurde nochmals soviel Kaliummethylat-Lösung hinzugefügt, daß eine entnommene homogenisierte Methanol/Salz-Mischung nach Verdünnen mit Wasser im Verhältnis 1:1 einen pH-Wert von 9,5 aufwies.
Nach Zugabe von 225,8 g Tetrabutylammoniumbromid und 10,0 kg 3-Chlorpropyl-trimethoxysilan wurde über eine kurze Kolonne solange Methanol abdestilliert, bis bei 118°C eine exotherme Reaktion einsetzte, die durch Kühlung auf einer Temperatur von unter 120°C gehalten wurde. Nach Abklingen der exothermen Reaktion wurde noch 2 h bei 120°C erwärmt. Aus der gekühlten Reaktionsmischung wurde das gebildete Kaliumchlorid abgeschleudert und mit Methanol gewaschen. Im Methanolfreien Filtrat wurde photometrisch ein Diimin-Gehalt von 2,8 g/kg ermittelt.
Nach Destillation über einen Kurzwegverdampfer (Typ KD 10 der Firma Leybold-Heraeus) unter einem Sauerstoff/Stickstoff-Strom (Volumenverhältnis 1:12) von 1750 ml/h, bei einer Verdampfertemperatur von 94 °C und einem Arbeitsdruck von 0,4 mbar, wurde eine Destillationsausbeute von 11,557 kg 93,2 % erreicht. Der photometrisch ermittelte Diimin-Gehalt betrug 92 mg/kg Destillat.
Nach Zugabe von 3,467 g 2,6-Di-tert.-butyl-4-methylphenol wurde über eine 40 cm lange Kolonne (Füllkörper V4A-Drahtnetze) unter einem O₂/N₂-Strom (Volumenverhältnis 1:11,9) von 150 l/h fraktioniert. Ausbeute: 11,146 kg (90,0 % d. Th.) 3-Methacryloxypropyltrimethoxysilan. Siedepunkt: 76-77°C/0,25 mbar. GC-Reinheit: 99,6 %.
Weder in den Destillationsrückständen noch im Kurzwegverdampfer oder der Destillationskolonne wurde "Popcorn"-Polymerisat festgestellt.

### Beispiel 3

In einem Rührreaktor wurden 9,807 kg einer 25,7 %igen. methanolischen Kaliummethylat-Lösung unter Kühlung mit 1,711 kg Acrylsäure versetzt und nach Zugabe von 45 g pulverförmigem N,N'-Diphenyl-p-phenylendiamin für 2 h bei rund 30 bis 40°C ein lebhafter Gasstrom, bestehend aus einem Sauerstoff/-Stickstoff-Gemisch (1:11,5), durch die Reaktionsmischung geleitet. Nach dieser Zeit betrug der photometrisch ermittelte Diamin-Umsatz 63 %. Nach Zugabe von weiteren 0,882 kg Acrylsäure und Entnahme einer Probe zeigte diese nach Verdünnung mit Wasser im Verhältnis 1:1 einen pH-Wert von 8,5 an.
Nach Zugabe von 163 g Tetrabutylammoniumbromid und 7,190 kg 3-Chlorpropyltrimethoxysilan wurde soweit Methanol über eine kurze Kolonne abdestilliert, daß bei 115°C eine exotherme Reaktion einsetzte. Nach deren Abklingen wurde 1,5 h bei 120°C weitererhitzt. Nach Abkühlung wurde vom gebildeten Kaliumchlorid abgeschleudert und mit Methanol gewaschen. Im vom Methanol befreiten Filtrat wurde photometrisch ein Diimin-Gehalt von 3,2 g/kg Filtrat bestimmt.
Nach Destillation über einen Kurzwegverdampfer (Typ KD 10 der Firma Leybold-Heraeus) wurden bei einer Verdampfertemperatur von 95°C und einem Arbeitsdruck von 0,4 mbar bei Einleitung eines Sauerstoff/Stickstoff-Gemisches (Volumenverhältnis 1:15) von 1.050 ml/h eine Destillationsausbeute von 94,3 % d. Th. erreicht. Der photometrisch ermittelte Diimin-Gehalt betrug 80 mg/kg Destillat.
Nach Zugabe von 1,9 g 2,6-Di-tert.-butyl-4-methylphenol wurde über eine 40 cm hohe Füllkörperkolonne unter o.g. Sauerstoff/Stickstoff-Verhältnis und einer eingeleiteten Gasmenge von 100 ml/h fraktioniert. Reinausbeute: 7,991 kg 89,5 % 3-Acryloxypropyltrimethoxysilan. Siedepunkt: 63-64 °C/0,1-0,15 mbar. GC-Reinheit: 99,3 %.
Weder in den Destillationsrückständen noch im Kurzwegverdampfer oder der Destillationskolonne war "Popcorn"-Polymerisat enthalten.

### Beispiel 4

In einem Mehrhalskolben mit Rührer, Tropftrichter, Thermometer und Kühler wurden 255 g Allylmethacrylat und 3 g 2,6-Di-tert.-butyl-4-methylphenol vorgelegt und unter Durchleiten eines schwachen Sauerstoff/Stickstoff-Gemisches (Volumenverhältnis 1:11,9) auf 105°C erhitzt. Aus dem Tropftrichter wurden 240 g Trimethoxysilan zugetropft. Nach einem Viertel, der Hälfte und drei Viertel der Silanzugabe sowie nach Beendigung der Silan-Zugabe wurden jeweils 250 mg einer 10 %igen acetonischen Lösung von H₂PtCl₆ hinzugefügt. Während der Reaktion wurde die Temperatur zwischen 100 und 105 °C gehalten. Nach 2 Stunden Reaktionsdauer wurde das erhaltene Rohprodukt unter Vakuum abgeflasht.
Nach Zugabe von 4,5 g N-Phenyl-N'-isopropyl-p-chinondiimin wurde der Rückstand unter reduziertem Druck über eine kurze Füllkörperkolonne destilliert, wobei als Hauptfraktion bei 110 bis 112°C (3 mbar) 417,6 g 3-Methacryloxypropyltrimethoxysilan übergingen, was einer Ausbeute von 85,6 %, bezogen auf eingesetztes Allylmethacrylat, entspricht.
In der Kolonne befand sich kein "Popcorn"-Polymerisat, der Destillationsrückstand war dünnflüssig, und das Destillat wies eine Hazenzahl von weniger als 10 auf.

### Vergleichsbeispiel A

Im Unterschied zu Beispiel 2 wurde hier so verfahren, daß anstelle von N,N'-Diphenyl-p-chinondiimin 4,2 g N,N'-Diphenyl-p-phenylendiamin unter weitgehendem Sauerstoffausschluß verwendet wurde. Das Flash-Destillat war fast farblos und enthielt weniger als 5 mg Diimin pro kg Destillat. Bei der Reindestillation über eine Kolonne ohne Verwendung eines sauerstoffhaltigen Schutzgases trat in erheblicher Menge "Popcorn"-Polymerisatbildung in der Kolonne und im Sumpf auf. Die Ausbeute lag bei 42 %.

### Vergleichsbeispiel B

Beispiel 2 wurde dahingehend geändert, daß anstelle von N,N'-Diphenyl-p-chinondiimin 4,2 g N,N'-Diphenyl-p-phenylendiamin und 0,23 g 2,6-Di-tert.-butyl-4-methylphenol sofort nach der Ausbildung von Kaliummethacrylat zugesetzt wurden. Die Reaktion wurde unter weitgehendem Sauerstoffausschluß durchgeführt. Das Flash-Destillat war praktisch farblos und wies weniger als 5 mg Diimin pro kg Destillat auf. Die GC-Analyse ergab keinen Hinweis auf 2.6-Di-tert.-butyl-4-methylphenol im Flash-Destillat. Bei der Reindestillation über eine Kolonne, ohne Verwendung eines sauerstoffhaltigen Schutzgases, trat in erheblicher Menge "Popcorn"-Polymerisatbildung in der Kolonne und im Sumpf auf. Die Ausbeute lag bei 39 %.

## Patentansprüche

1. Verwendung von N,N'-disubstituierten p-Chinondiiminen der allgemeinen Formel I 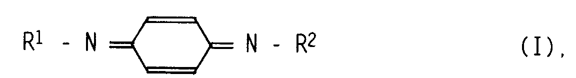 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, zur Stabilisierung von Methacryloxy- oder Acryloxygruppen enthaltenden Organosilanen der allgemeinen Formel II 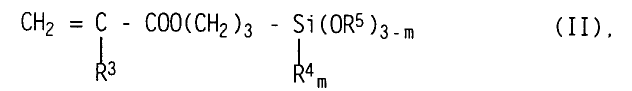 in der R³ für ein Wasserstoffatom oder eine Methyl-Gruppe steht und R⁴ und R⁵ gleiche oder verschiedene Alkyl-Gruppen mit 1 bis 4 C-Atomen sind und m gleich 0 oder 1 oder 2 ist.

2. Zusammensetzung, bestehend aus Methacryloxy- oder Acryloxy-Gruppen enthaltenden Organosilanen der allgemeinen Formel II 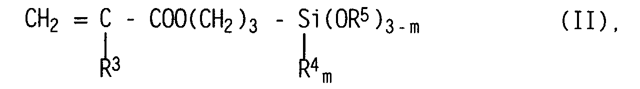 in der R³ für ein Wasserstoffatom oder eine Methyl-Gruppe steht und R⁴ und R⁵ gleiche oder verschiedene Alkyl-Gruppen mit 1 bis 4 C-Atomen sind und m gleich 0 oder 1 oder 2 ist, und einem oder mehreren N,N'-disubstituierten p-Chinondiiminen der allgemeinen Formel I 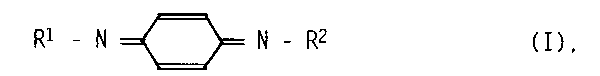 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, als Stabilisator(en).

3. Zusammensetzung nach Anspruch 2, dadurch gekennzeichnet, daß der Gehalt an p-Chinondiiminen der allgemeinen Formel I >0,0005 bis 1,5 Gew.-% beträgt.

4. Zusammensetzung nach Anspruch 3, dadurch gekennzeichnet, daß der Gehalt an p-Chinondiiminen der allgemeinen Formel I 0,01 bis 1,0 Gew.-% beträgt.

5. Zusammensetzung nach Anspruch 4, dadurch gekennzeichnet, daß der Gehalt der an p-Chinondiiminen der allgemeinen Formel I 0,1 bis 0,5 Gew.-% beträgt.

6. Zusammensetzung nach den Ansprüchen 2 bis 5, dadurch gekennzeichnet, daß als zusätzliche Stabilisatoren N,N'-disubstituierte p-Phenylendiamine der allgemeinen Formel IV 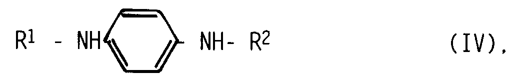 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, und/oder flüchtige, sterisch gehinderte Phenole der allgemeinen Formel V 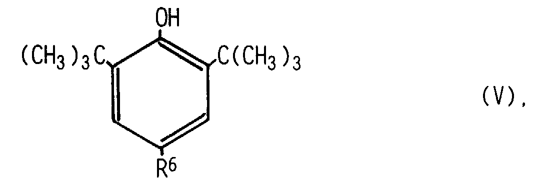 worin R⁶ eine Alkyl- oder Alkoxy-Gruppe mit 1 bis 4 C-Atomen ist, enthalten sind.

7. Verfahren zur Stabilisierung von Methacryloxy- oder Acryloxy-Gruppen enthaltenden Organosilanen der allgemeinen Formel II 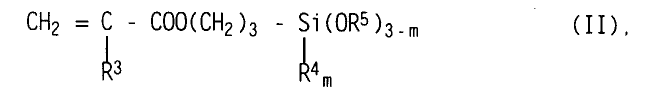 in der R³ für ein Wasserstoffatom oder eine Methyl-Gruppe steht und R⁴ und R⁵ gleiche oder verschiedene Alkyl-Gruppen mit 1 bis 4 C-Atomen sind und m gleich 0 oder 1 oder 2 ist, dadurch gekennzeichnet, daß man als Stabilisatoren N,N'-disubstituierte p-Chinondiimine der allgemeinen Formel I 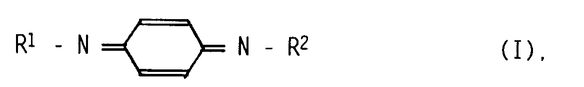 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, einsetzt.

8. Verfahren nach Anspruch 7, dadurch gekennzeichnet, daß die als Stabilisatoren eingesetzten p-Chinondiimine der allgemeinen Formel I während des Herstellprozesses der Organosilane der allgemeinen Formel II zugesetzt werden.

9. Verfahren nach Anspruch 7, dadurch gekennzeichnet, daß die als Stabilisatoren eingesetzten p-Chinondiimine der allgemeinen Formel I während des Herstellprozesses der Organosilane der allgemeinen Formel II erzeugt werden.

10. Verfahren nach Anspruch 9, dadurch gekennzeichnet, daß die p-Chinondiimine der allgemeinen Formel I durch Oxidation der korrespondierenden p-Phenylendiamine der allgemeinen Formel IV erzeugt werden.

11. Verfahren nach Anspruch 10, dadurch gekennzeichnet, daß als Oxidationsmittel Sauerstoff im Gemisch mit Stickstoff verwendet wird.

12. Verfahren nach den Ansprüchen 9 bis 11, dadurch gekennzeichnet, daß die Oxidation bei einer Temperatur zwischen 10 und 60 °C durchgeführt wird.

13. Verfahren nach Anspruch 12, dadurch gekennzeichnet, daß die Oxidation bei einer Temperatur zwischen 20 und 40 °C durchgeführt wird.

14. Verfahren nach Anspruch 13, dadurch gekennzeichnet, daß die Oxidation bei einer Temperatur zwischen 25 und 35 °C durchgeführt wird.

15. Verfahren nach den Ansprüchen 9 bis 14, dadurch gekennzeichnet, daß die p-Chinondiimine der allgemeinen Formel I durch die Oxidation der korrespondierenden p-Phenylendiamine der allgemeinen Formel IV im Reaktionsmilieu der Stufe 1 des PTC-Verfahrens erzeugt werden.

16. Verfahren nach den Ansprüchen 7 bis 15, dadurch gekennzeichnet, daß die Roh-Organosilane der allgemeinen Formel II >0,0005 bis 1,5 Gew.-% p-Chinondiimine der allgemeinen Formel I enthalten.

17. Verfahren nach Anspruch 16, dadurch gekennzeichnet, daß die Roh-Organosilane der allgemeinen Formel II 0,01 bis 1,0 Gew.-% p-Chinondiimine der allgemeinen Formel I enthalten.

18. Verfahren nach Anspruch 17, dadurch gekennzeichnet, daß die Roh-Organosilane der allgemeinen Formel II 0,1 bis 0,5 Gew.-% p-Chinondiimine der allgemeinen Formel I enthalten.

19. Verfahren nach den Ansprüchen 7 bis 18, dadurch gekennzeichnet, daß man als zusätzliche Stabilisatoren N,N'-disubstituierte p-Phenylendiamine der allgemeinen Formel IV 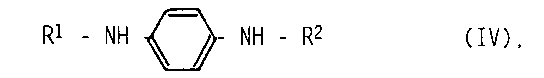 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, und/oder flüchtige, sterisch gehinderte Phenole der allgemeinen Formel V 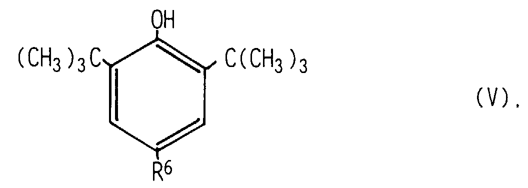 worin R⁶ eine Alkyl- oder Alkoxy-Gruppe mit 1 bis 4 C-Atomen ist, einsetzt.

20. Phasentransferkatalyse-Verfahren (PTC-Verfahren) zur Herstellung von Methacryloxy- oder Acryloxy-Gruppen enthaltenden Organosilanen der allgemeinen Formel II 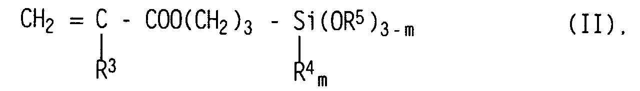 in der R³ für ein Wasserstoffatom oder eine Methyl-Gruppe steht und R⁴ und R⁵ gleiche oder verschiedene Alkyl-Gruppen mit 1 bis 4 C-Atomen sind und m gleich 0 oder 1 oder 2 ist, dadurch gekennzeichnet, daß die Roh-Organosilane der allgemeinen Formel II während des PTC-Verfahrens durch N,N'-disubstituierte p-Chinondiimine der allgemeinen Formel I 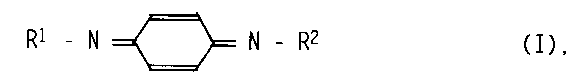 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, stabilisiert werden.

21. Verfahren nach Anspruch 20, dadurch gekennzeichnet, daß während des PTC-Verfahrens die als Stabilisatoren verwendeten p-Chinondiimine der allgemeinen Formel I zugesetzt und/oder erzeugt werden.

22. Verfahren nach den Ansprüchen 20 und 21, dadurch gekennzeichnet, daß die Roh-Organosilane der allgemeinen Formel II, die p-Chinondiimine der allgemeinen Formel I enthalten, zur Abtrennung von Hochsiedern einer Flash-Destillation unterzogen werden.

23. Verfahren nach Anspruch 22, dadurch gekennzeichnet, daß das Flash-Destillat mit einer Menge von > 5 bis 15.000 Gew.-ppm p-Chinondiiminen der allgemeinen Formel I stabilisiert wird.

24. Verfahren nach Anspruch 23, dadurch gekennzeichnet, daß das Flash-Destillat mit einer Menge von 10 bis 3.000 Gew.-ppm p-Chinondiiminen der allgemeinen Formel I stabilisiert wird.

25. Verfahren nach Anspruch 24, dadurch gekennzeichnet, daß das Flash-Destillat mit einer Menge von 50 bis 1 000 Gew.-ppm p-Chinondiiminen der allgemeinen Formel I stabilisiert wird.

26. Verfahren nach den Ansprüchen 20 bis 25, dadurch gekennzeichnet, daß das Flash-Destillat fraktioniert destilliert wird.

27. Verfahren nach Anspruch 26, dadurch gekennzeichnet, daß die über Kopf der Kolonne destillierten Methacryloxy- oder Acryloxy-Gruppen enthaltenden Organosilane frei von p-Chinondiiminen der allgemeinen Formel I erhalten werden.

28. Verfahren nach den Ansprüchen 20 bis 27, dadurch gekennzeichnet, daß die p-Chinondiimine der allgemeinen Formel I und N,N'-disubstituierte p-Phenylendiamine der allgemeinen Formel IV 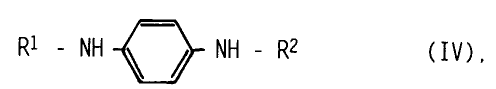 worin R¹ und R² gleich oder verschieden sind und eine Phenyl-Gruppe und/oder eine geradkettige und/oder verzweigte Alkyl-Gruppe mit 3 bis 8 C-Atomen und/oder eine Cyclohexyl-Gruppe bedeuten, dem Eduktgemisch und/oder dem Reaktionsgemisch und/oder dem Roh-Organosilan der allgemeinen Formel II und/oder dem Flash-Destillat und/oder an einer anderen Stelle der destillativen Aufarbeitung zugegeben werden.

29. Verfahren nach Anspruch 28, dadurch gekennzeichnet, daß dem Roh-Organosilan der allgemeinen Formel II und/oder dem Flash-Destillat und/oder an einer anderen Stelle der destillativen Aufarbeitung und/oder dem Reindestillat flüchtige, sterisch gehinderte Phenole der allgemeinen Formel V 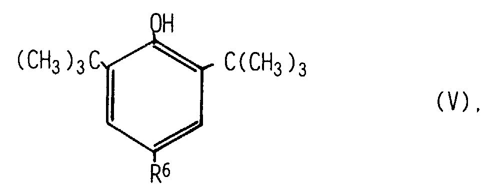 worin R⁶ eine Alkyl- oder Alkoxy-Gruppe mit 1 bis 4 C-Atomen ist. zugegeben werden.

30. Verfahren nach den Ansprüchen 20 bis 29, dadurch gekennzeichnet, daß den Methacryloxy- oder Acryloxy-Gruppen enthaltenden Organosilanen der allgemeinen Formel II nach fraktionierter Destillation ein nicht färbendes, sterisch gehindertes Phenol der allgemeinen Formel V zur Nachstabilisierung zugegeben wird.

31. Verfahren nach den Ansprüchen 29 und 30, dadurch gekennzeichnet, daß 2,6-Di-tert.-butyl-4-methylphenol zugegeben wird.

## Claims

1. The use of N,N'-disubstituted p-quinonediimines of the general formula I 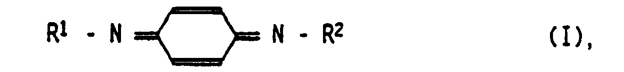 where R¹ and R² are identical or different and are each a phenyl group and/or a straight-chain and/or branched alkyl group having from 3 to 8 carbon atoms and/or a cyclohexyl group, for stabilizing organosilanes containing methacryloxy or acryloxy groups and having the general formula II 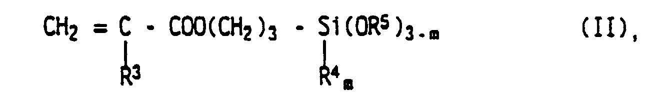 where R³ is a hydrogen atom or a methyl group and R⁴ and R⁵ are identical or different alkyl groups having from 1 to 4 carbon atoms and m is equal to 0 or 1 or 2.

2. A composition comprising organosilanes containing methacryloxy or acryloxy groups and having the general formula II 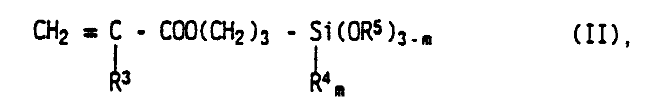 where R³ is a hydrogen atom or a methyl group and R⁴ and R⁵ are identical or different alkyl groups having from 1 to 4 carbon atoms and m is equal to 0 or 1 or 2, and as stabilizer one or more N,N'-disubstituted p-quinonediimines of the general formula I 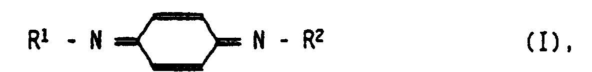 where R¹ and R² are identical or different and are each a phenyl group and/or a straight-chain and/or branched alkyl group having from 3 to 8 carbon atoms and/or a cyclohexyl group.

3. A composition according to claim 2,
characterized in that the content of p-quinonediimines of the general formula I > 0.0005 to 1.5% by weight.

4. A composition according to claim 3,
characterized in that the content of p-quinonediimines of the general formula I is from 0.01 to 1.0% by weight.

5. A composition according to claim 4,
characterized in that the content of p-quinonediimines of the general formula I is from 0.1 to 0.5% by weight.

6. A composition according to any of claims 2 to 5,
characterized in that N,N'-disubstituted p-phenylenediamines of the general formula IV 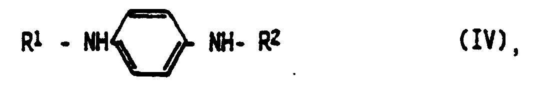 where R¹ and R² are identical or different and are each a phenyl group and/or a straight-chain and/or branched alkyl group having from 3 to 8 carbon atoms and/or a cyclohexyl group, and/or volatile, sterically hindered phenols of the general formula V 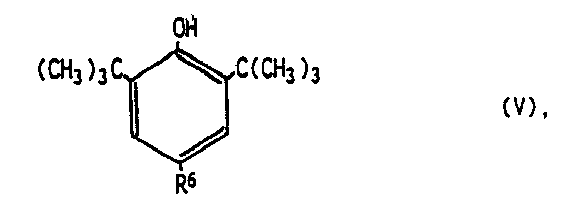 where R⁶ is an alkyl or alkoxy group having from 1 to 4 carbon atoms, are present as additional stabilizers.

7. A process for stabilizing organosilanes containing methacryloxy or acryloxy groups and having the general formula II 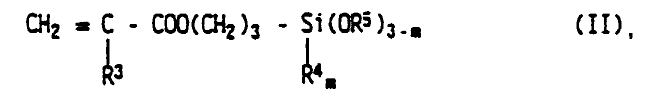 where R³ is a hydrogen atom or a methyl group and R⁴ and R⁵ are identical or different alkyl groups having from 1 to 4 carbon atoms and m is equal to 0 or 1 or 2,
characterized in that N,N'-disubstituted p-quinonediimines of the general formula I 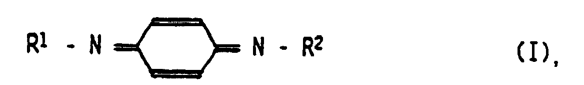 where R¹ and R² are identical or different and are each a phenyl group and/or a straight-chain and/or branched alkyl group having from 3 to 8 carbon atoms and/or a cyclohexyl group, are used as stabilizers.

8. A process according to claim 7,
characterized in that the p-quinonediimines of the general formula I used as stabilizers are added during the preparation process of the organosilanes of the general formula II.

9. A process according to claim 7,
characterized in that the p-quinonodiimines of the general formula I used as stabilizers are generated during the preparation process of the organosilanes of the general formula II.

10. A process according to claim 9,
characterized in that the p-quinonediimines of the general formula I are produced by oxidation of the corresponding p-phenylenediamines of the general formula IV.

11. A process according to claim 10,
characterized in that oxygen in admixture with nitrogen is used as oxidizing agent.

12. A process according to any of claims 9 to 11,
characterized in that the oxidation is carried out at a temperature of from 10 to 60°C.

13. A process according to claim 12,
characterized in that the oxidation is carried out at a temperature of from 20 to 40°C.

14. A process according to claim 13,
characterized in that the oxidation is carried out at a temperature of from 25 to 35°C.

15. A process according to any of claims 9 to 14,
characterized in that the p-quinonediimines of the general formula I are produced by the oxidation of the corresponding p-phenylenediamines of the general formula IV in the reaction medium of stage 1 of the PTC process.

16. A process according to any of claims 7 to 15,
characterized in that the crude organosilanes of the general formula II contain from > 0.0005 to 1.5% by weight of p-quinonediimines of the general formula I.

17. A process according to claim 16,
characterized in that the crude organosilanes of the general formula II contain from 0.01 to 1.0% by weight of p-quinonediimines of the general formula I.

18. A process according to claim 17,
characterized in that the crude organosilanes of the general formula II contain from 0.1 to 0.5% by weight of p-quinonediimines of the general formula I.

19. A process according to any of claims 7 to 18,
characterized in that N,N'-disubstituted p-phenylenediamines of the general formula IV 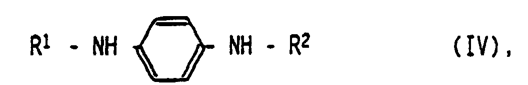 where R¹ and R² are identical or different and are each a phenyl group and/or a straight-chain and/or branched alkyl group having from 3 to 8 carbon atoms and/or a cyclohexyl group, and/or volatile, sterically hindered phenols of the general formula V 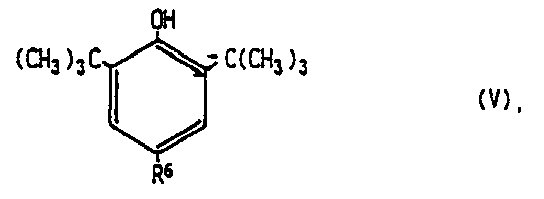 where R⁶ is an alkyl or alkoxy group having from 1 to 4 carbon atoms, are used as additional stabilizers.

20. A phase-transfer catalysis process (PTC process) for preparing organosilanes containing methacryloxy or acryloxy groups and having the general formula II 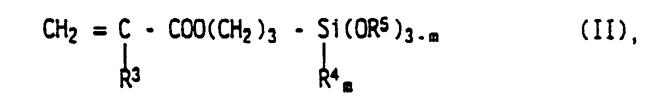 where R³ is a hydrogen atom or a methyl group and R⁴ and R⁵ are identical or different alkyl groups having from 1 to 4 carbon atoms and m is equal to 0 or 1 or 2,
characterized in that the crude organosilanes of the general formula II are stabilized during the PTC process by N,N'-disubstituted p-quinonediimines of the general formula I 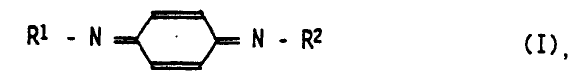 where R¹ and R² are identical or different and are each a phenyl group and/or a straight-chain and/or branched alkyl group having from 3 to 8 carbon atoms and/or a cyclohexyl group.

21. A process according to claim 20,
characterized in that the p-quinonediimines of the general formula I used as stabilizers are added and/or generated during the PTC process.

22. A process according to either of claims 20 and 21,
characterized in that the crude organosilanes of the general formula II containing the p-quinonediimines of the general formula I are subjected to a flash distillation to separate off high boilers.

23. A process according to claim 22,
characterized in that the flash distillate is stabilized with an amount of from > 5 to 15,000 ppm by weight of p-quinonediimines of the general formula I.

24. A process according to claim 23,
characterized in that the flash distillate is stabilized with an amount of from 10 to 3,000 ppm by weight of p-quinonediimines of the general formula I.

25. A process according to claim 24,
characterized in that the flash distillate is stabilized with an amount of from 50 to 1,000 ppm by weight of p-quinonediimines of the general formula I.

26. A process according to any of claims 20 to 25,
characterized in that the flash distillate is fractionally distilled.

27. A process according to claim 26,
characterized in that the organosilanes containing methacryloxy or acryloxy groups distilled over via the top of the column are obtained free of p-quinonediimines of the general formula I.

28. A process according to any of claims 20 to 27,
characterized in that the p-quinonediimines of the general formula I and N,N'-disubstituted p-phenylenediamines of the general formula IV 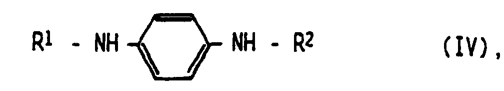 where R¹ and R² are identical or different and are each a phenyl group and/or a straight-chain and/or branched alkyl group having from 3 to 8 carbon atoms and/or a cyclohexyl group, are added to the starting material mixture and/or the reaction mixture and/or the crude organosilane of the general formula II and/or the flash distillate and/or at another point in the distillative workup.

29. A process according to claim 28,
characterized in that volatile, sterically hindered phenols of the general formula V 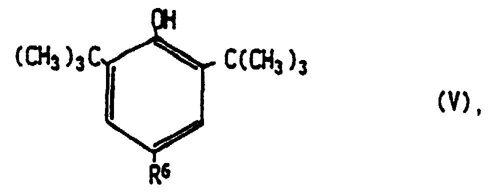 where R⁶ is an alkyl or alkoxy group having from 1 to 4 carbon atoms, are added to the crude organosilane of the general formula II and/or the flash distillate and/or at another point in the distillative workup and/or to the pure distillate.

30. A process according to any of claims 20 to 29,
characterized in that a non-colouring, sterically hindered phenol of the general formula V is added for post-stabilization to the organosilanes containing methacryloxy or acryloxy groups and having the general formula II after fractional distillation.

31. A process according to either of claims 29 and 30,
characterized in that 2,6-di-tert-butyl-4-methylphenol is added.

## Revendications

1. Utilisation de p-quinone diimines N,N'-disubstituées de formule générale I 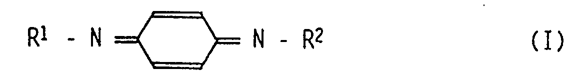 dans laquelle R¹ et R² sont identiques ou différents et représentent un groupe phényle et/ou un groupe alkyle à chaîne droite et/ou ramifiée ayant de 3 à 8 atomes de carbone et/ou un groupe cyclohexyle, pour la stabilisation d'organosilanes contenant des groupes méthacryloxy ou acryloxy de formule générale II 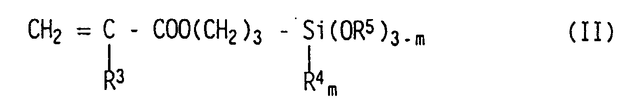 dans laquelle R³ représente un atome d'hydrogène ou un groupe méthyle et R⁴ et R⁵ représentent des groupes alkyle identiques ou différents ayant de 1 à 4 atomes de carbone et m vaut 0 ou 1 ou 2.

2. Composition constituée d'organosilanes contenant des groupes méthacryloxy ou acryloxy de formule générale II 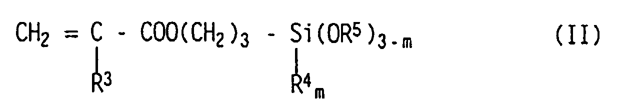 dans laquelle R³ représente un atome d'hydrogène ou un groupe méthyle et R⁴ et R⁵ représentent des groupes alkyle identiques ou différents ayant de 1 à 4 atomes de carbone et m vaut 0 ou 1 ou 2, et d'une ou plusieurs p-quinones diimines N,N'-disubstituées de formule générale I 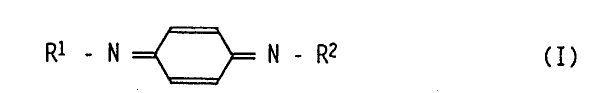 dans laquelle R¹ et R² sont identiques ou différents et représentent un groupe phényle et/ou un groupe alkyle à chaîne droite et/ou ramifiée ayant de 3 à 8 atomes de carbone et/ou un groupe cyclohexyle comme stabilisateur(s).

3. Composition selon la revendication 2,
caractérisé en ce que
la teneur en p-quinone diimines de formule générale I est comprise de plus 0,0005 à 1,5 % en poids.

4. Composition selon la revendication 3,
caractérisée en ce que
la teneur en p-quinone diimines de formule générale I est comprise de 0,01 à 1,0 % en poids.

5. Composition selon la revendication 4,
caractérisée en ce que
la teneur en p-quinone diimines de formule générale I est comprise de 0,1 à 0,5 % en poids.

6. Composition selon les revendications 2 à 5,
caractérisée en ce qu'
elle contient comme stabilisateurs supplémentaires des p-phénylène diamines N,N'-disubstituées de formule générale IV 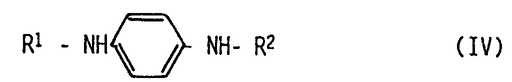 dans laquelle R¹ et R² sont identiques ou différents et représentent un groupe phényle et/ou un groupe alkyle à chaîne droite et/ou ramifiée ayant de 3 à 8 atomes de carbone et/ou un groupe cyclohexyle, et/ou des phénols volatils stériquement empêchés de formule générale V 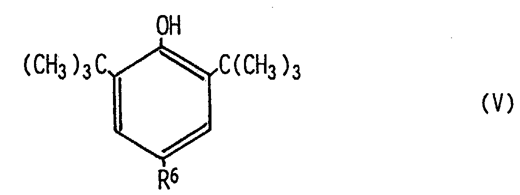 dans laquelle R⁶ représente un groupe alkyle ou alcoxy ayant de 1 à 4 atomes de carbone.

7. Procédé de stabilisation d'organosilanes contenant des groupes méthacryloxy ou acryloxy de formule générale II 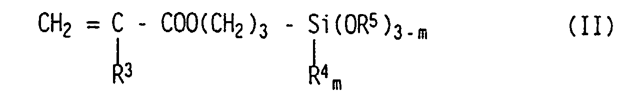 dans laquelle R³ représente un atome d'hydrogène, ou un groupe méthyle et R⁴ et R⁵ représentent des groupes alkyle identiques ou différents ayant de 1 à 4 atomes de carbone, et m vaut 0 ou 1 ou 2,
caractérisé en ce qu'on utilise comme stabilisateurs des p-quinone diimines N,N'-disubstituées de formule générale I 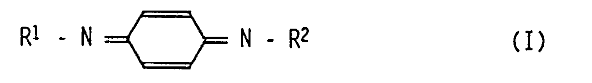 dans laquelle R¹ et R² sont identiques ou différents et représentent un groupe phényle et/ou un groupe alkyle à chaîne droite et/ou ramifiée ayant de 3 à 8 atomes de carbone et/ou un groupe cyclohexyle.

8. Procédé selon la revendication 7,
caractérisé en ce qu'
on ajoute les p-quinones diimines de formule générale I utilisées comme stabilisateur au cours du procédé de fabrication des organosilanes de formule générale II.

9. Procédé selon la revendication 7,
caractérisé en ce qu'
on produit les p-quinones diimines de formule générale I utilisées comme stabilisateurs au cours du procédé de fabrication des organosilanes de formule générale II.

10. Procédé selon la revendication 9,
caractérisé en ce qu'
on produit les p-quinones diimines de formule générale I par oxydation des p-phénylène diamines de formule générale IV correspondantes.

11. Procédé selon la revendication 10,
caractérisé en ce qu'
on utilise comme oxydant l'oxygène mélangé à l'azote.

12. Procédé selon les revendications 9 à 11,
caractérisé en ce qu'
on conduit l'oxydation à une température comprise entre 10 et 60°C.

13. Procédé selon la revendication 12,
caractérisé en ce qu'
on conduit l'oxydation à une température comprise entre 20 et 40°C.

14. Procédé selon la revendication 13,
caractérisé en ce qu'
on conduit l'oxydation à une température comprise entre 25 et 35°C.

15. Procédé selon les revendications 9 à 14,
caractérisé en ce qu'
on produit les p-quinone diimines de formule générale I par oxydation des p-phénylène diamines de formule générale IV correspondantes dans le milieu réactionnel de l'étape I du procédé CTP.

16. Procédé selon les revendications 7 à 15,
caractérisé en ce que les organosilanes bruts de formule générale II contiennent plus de 0,0005 à 1,5 % en poids de p-quinone diimines de formule générale I.

17. Procédé selon la revendication 16,
caractérisé en ce que
les organosilanes bruts de formule générale II contiennent 0,01 à 1,0 % en poids de p-quinone diimines de formule générale I.

18. Procédé selon la revendication 17,
caractérisé en ce que
les organosilanes bruts de formule générale II contiennent 0,1 à 0,5 % en poids de p-quinone diimines de formule générale I.

19. Procédé selon les revendications 7 à 18,
caractérisé en ce qu'
on utilise comme stabilisateurs supplémentaires des p-phénylène diamines N,N-disbsubstituées de formule générale IV 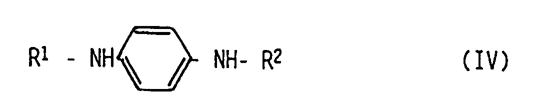 dans laquelle R¹ et R² sont identiques ou différents et représentent un groupe phényle et/ou un groupe alkyle à chaîne droite et/ou ramifiée ayant de 3 à 8 atomes de carbone et/ou un groupe cyclohexyle, et/ou des phénols volatils stériquement empêchés de formule générale V 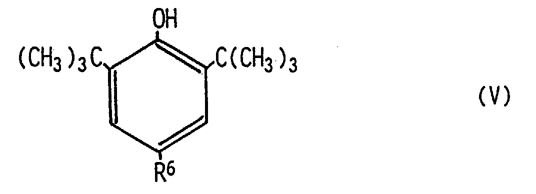 dans laquelle R⁶ représente un groupe alkyle ou alcoxy ayant de 1 à 4 atomes de carbone.

20. Procédé de catalyse à transfert de phase (procédé CTP) de fabrication d'organosilanes contenant des groupes méthacryloxy ou acryloxy de formule générale II 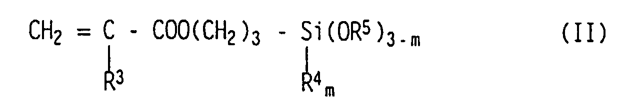 dans laquelle R³ représente un atome d'hydrogène ou un groupe méthyle et R⁴ et R⁵ représentent des groupes alkyle identiques ou différents ayant de 1 à 4 atomes de carbone et m vaut 0 ou 1 ou 2,
caractérisé en ce qu'
on stabilise les organosilanes bruts de formule générale II au cours du procédé CTP par des p-quinone diimines N,N-disubstituées de formule générale I 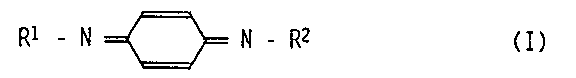 dans laquelle R¹ et R² sont identiques ou différents et représentent un groupe phényle et/ou un groupe alkyle à chaîne droite et/ou ramifiée ayant de 3 à 8 atomes de carbone et/ou un groupe cyclohexyle.

21. Procédé selon la revendication 20,
caractérisé en ce qu'
au cours du procédé CTP on ajoute et/ou on produit les p-quinone diimines de formule générale I utilisées comme stabilisateurs.

22. Procédé selon les revendications 20 et 21,
caractérisé en ce qu'
on soumet à une distillation instantanée les organosilanes bruts de formule générale II qui contiennent les p-quinone diimines de formule générale I aux fins de séparation des matières à haut point d'ébullition.

23. Procédé selon la revendication 22,
caractérisé en ce qu'
on stabilise le produit de distillation instantané avec une quantité supérieure à 5 à 15 000 ppm en poids de p-quinone diimines de formule générale I.

24. Procédé selon la revendication 23,
caractérisé en ce qu'
on stabilise le produit de distillation instantané avec une quantité de 10 à 3000 ppm de poids de p-quinone diimines de formule générale I.

25. Procédé selon la revendication 24,
caractérisé en ce qu'
on stabilise le produit de distillation instantané avec une quantité de 50 à 1000 ppm en poids de p-quinone diimines de formule générale I.

26. Procédé selon les revendications 20 à 25,
caractérisé en ce qu'
on distille le produit de distillation instantané de façon fractionnée.

27. Procédé selon la revendication 26,
caractérisé en ce qu'
on obtient les organosilanes contenant des groupes méthacryloxy ou acryloxy par la tête de la colonne sous forme dépourvue de p-quinone diimines de formule générale I.

28. Procédé selon les revendications 20 à 27,
caractérisé en ce qu'
on ajoute les p-quinone diimines de formule générale I et les p-phénylène diamines N,N'-disubstituées de formule générale IV 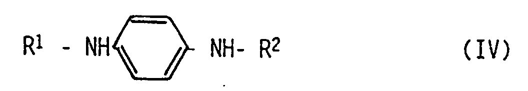 dans lesquelles R¹ et R² sont identiques ou différents et représentent un groupe phényle et/ou un groupe alkyle à chaîne droite et/ou ramifiée ayant de 3 à 8 atomes de carbone et/ou un groupe cyclohexyle, au mélange de l'éduit et/ou au mélange réactionnel et/ou à l'organosilane brut de formule générale I et/ou au produit de distillation instantané et/ou en un autre endroit du traitement de distillation.

29. Procédé selon la revendication 28,
caractérisé en ce qu'
on ajoute à l'organosilane brut de formule générale II et/ou au produit de distillation instantané et/ou en un autre endroit du traitement de distillation et/ou au produit de distillation brut des phénols volatils stériquement empêchés de formule générale V 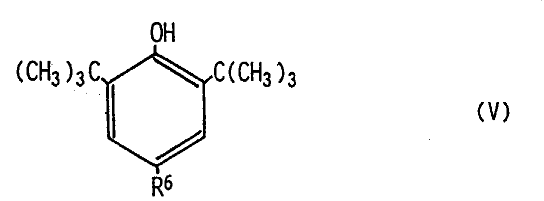 dans laquelle R⁶ représente un groupe alkyle ou alcoxy ayant de 1 à 4 atomes de carbone.

30. Procédé selon les revendications 1 à 29,
caractérisé en ce qu'
on ajoute aux organosilanes contenant des groupes méthacryloxy ou acryloxy de formule générale II, après distillation fractionnée, un phénol stêriquement empêché non colorant de formule générale V aux fins de stabilisation ultérieure.

31. Procédé selon les revendications 29 et 30,
caractérisé en ce qu'
on ajoute le 2,6-di-tert.butyl-4-méthylphénol.
